# EUROPEAN PATENT SPECIFICATION

(11) **EP 2 916 741 B1**
(45) Date of publication and mention of the grant of the patent: **21.03.2018**
(21) Application number: 13829003.6
(22) Date of filing: 11.11.2013
(51) Int. Cl.: A61B 17/00

(54) **MEDICAL DEVICE FOR THE TREATMENT OF ANAL FISTULA**
MEDIZINISCHE VORRICHTUNG ZUR BEHANDLUNG VON ANALFISTELN
DISPOSITIF MÉDICAL POUR LE TRAITEMENT DE FISTULE ANALE

(30) Priority: 09.11.2012 IT PD20120336
(43) Date of publication of application: 16.09.2015
(73) Proprietor: Bertoli, Alice, 35031 Abano Terme (PD) (IT)
(72) Inventor: Bertoli, Alice, 35031 Abano Terme (PD) (IT)
(86) International application number: PCT/IT2013/000311
(87) International publication number: WO 2014/073015

(56) References cited:
- US-A1- 2009 125 119
- US-A1- 2010 256 777
- US-A1- 2012 035 644

## Description

The present invention relates to a medical device for the treatment of anal fistulas.

The anal fistula is a tunnel which forms in the subcutaneous plane between the anal sphincter gland and the perianal skin. It arises from glandular infection that makes its way into internal tissues and the perianal area leading outside the body.

Fistulas are classified according to their position and the relationship that they have with the anal sphincters:
Lower transphincteric anal fistulas: when the fistula route crosses throughout the subcutaneous plane and the lower end of the sphincter muscle ; these are the most frequent (about 63% of all anal fistulas).
Higher transphincteric anal fistulas : when the fistula route crosses the whole subcutaneous plane and passes through the external sphincter (about 27% of all anal fistulas).
Suprasphinteric anal fistulas: where the route crosses the entire external sphincter and part or all of the puborectalis muscle (about 4%).

Intersphincteric (or intramural) anal fistulas are fistulas in which the route is located in the Eisenhammer intersphincteric groove, ie between the internal sphincter and the external sphincter (about 6%). These fistulas also have their origin at the level of the dentate line but unlike the others, they have evolved in an ascending direction. Occasionally fistulas can open in the rectum-anorectal fistulas.

The most up to date surgeries possible for the treatment of anal fistulas are substantially the following:
1. The Fistulotomy. This is the surgical opening of the fistulous tract. This method is performed in more simple fistulas and gives good results. A simple fistula is a fistula which is easy to treat : the fistulous tract is easily identified and anorectal function is not significantly impaired by surgery. Non-complex intersphincteric fistulas and Lower transphincteric anal fistulas belong to this category.
2. The fistulectomy consists of the dissection of the entire fistula comprising several millimeters of surrounding healthy tissue. The surgical wound is left open so that it can heal by secondary intention.
3. The two-part fistulectomy is required in the treatment of transphincteric fistulas, ie which cross the external sphincter muscle and also of suprasphincteric and complex fistulas. The first intervention involves the retrograde dissection from the orifice of the fistula up to the external muscular plane, flattening the path of the fistula and positioning a cutting seton. The role of the seton is to slowly divide the sphincter apparatus thus avoiding sphincter damage that could compromise the corrective surgery. During the second intervention the section of the previously treated muscular plane has given way to a solid healing. The fistulectomy can then be carried out. Fistulotomy and fistulectomy interventions offer and excellent recovery rate and if the initial process of diagnostic therapy is also correct there is a very low recurrence rate.
4. " Advancement flap" : This is an intervention that involves a running fistulectomy (removal of the entire fistula), repair of the breached sphincter and coverage ( from the inside of the anal canal) of the muscle through using an " advancement flap" , which is an elastic strip of mucosa and submucosa removed from the rectum above, that is attached to cover the surgery site and previous fistula orifice. This prevents the entry of faeces into the fistula canal and allows the muscles to be saved.
5. Treatment by injection of fibrin glue (made from coagulant substances present in human serum) into the fistula. In this case the integrity of the muscle tissue is not compromised, but this treatment has a high relapse rate (over 50%).
6. Plug: this technique involves the insertion of an "anal plug " (literally " anal plug ") cone-shaped, rat-tail, derived from the submucosa of pig intestine (eg Surgisis ANAL FISTULA PLUG), properly treated to make it biocompatible . Or it can be formed by a series of pipes connected to a circular disk in a bioabsorbable material such as polyglycolic acid (example GORE B10 -A PLUG). The plug is inserted into the fistula and acts as a support for the healing tissues of the patient. This method , while having the advantage of maintaining the condition of the muscles, has not, however, a high percentage of success, as the medical device can be easily extruded from the body.

The current surgical techniques, associated with known devices, therefore, are not decisive of the pathology, always presenting high percentages of failure.

With relation to the incorporation of biomaterial implants into the human body , from a biomechanical point of view, the mechanisms which they induce are noted:
1. Natural process of integration (if it is inert biomaterial) or remodeling (if it is bioactive biomaterial) The integration of the inert biomaterial (synthetic origin) allows the healing of the damaged human tissue , despite being accepted as a foreign structure it is compatible with the human body; while the integration of bioactive biomaterial (animal) allows healing of the damaged tissue which recognizes the bioactive biomaterial as part of itself that reshapes, making it part of its fabric.
2. Mechanical conditions which must be present for the system of integration or remodeling to occur: primary stability and intimate contact:
   a) PRIMARY STABILITY: the implanted device must not move within the fistulous tract. It is well known that in the case of damage to tissue such as bone, it is important to immobilize the two abutments of the fracture in order to promote healing. The implanted device requires the same primary stability.
   b) intimate contact with vascular structures : the presence of blood flow. It is obvious that if the elements necessary for tissue repair (platelets, growth factors, fibroclasts, fibroblasts, etc..) are found in blood, It is the intimate contact between the latter and the device which determines its integration as a support to the repair of the damaged tissue.

The devices currently in use for the treatment of anal fistulas do not meet the above conditions.

Indeed they are cylindrical or truncated cones without self-stabilizing surface macrostructures on the rotary and axial planes and they are hollow.

The geometric shape of the truncated cone as well as the cylinder does not prevent , indeed encourages, the rotary movement on the longitudinal axis. The cylindrical form also does not even prevent displacement in the axial direction.

The hollow body of the cylinder or truncated cone facilitates its collapse when depressed by external forces. These geometrical shapes are not optimal for the mechanical conditions for integration or remodeling of a medical device because they do not guarantee either the primary stability of the implant which can rotate on itself or to allow axial dislocation, nor the intimate contact with the vascular walls of the fistulous tract as they are collapsible under pressure from external muscular forces. This is the reason for which the new device was designed; for the surgical treatment of anal fistulas (plug) which is innovative due to its particular characteristics which comply with the principles of advanced biomechanics.

The objective for the subject of the present finding is, therefore to solve the technical problems, eliminating the drawbacks of medical devices known today.

The new device for the treatment of anal fistula acts as a stopper (plug) respecting the same biomechanical conditions mentioned above: primary stability and intimate contact, and this in way can be integrated into or remodeled by the body thus promoting healing.

This is a device made in surgically implantable biomaterial.

The invention has geometrical characteristics that allow both the primary stability and the intimate contact with the walls of the fistula as the medical device has sharp edges along the longitudinal axis to prevent rotation on its axis, being in the shape of prism, in order to present slipping. Secondly, the device has a solid body and therefore is not collapsible even if subjected to external forces. On the contrary, the solid body of the invention inserts "pressure "(press-fit) into the fistulous tract, forcing against the walls , thus determining the condition of intimate contact with the vascularized tissue.

The invention thus conceived is already stable, but can also be provided with pairs of notches in the body of the device. These incisions will be closed during the act of insertion of the medical device in the fistulous tract, they will open and thus oppose the extrusion in the case of movement against the device in the direction of the fistulous tract from which it was inserted (into the thickest part of the tract).

The distal tip of the medical device is fixed by a suture which, along with axial tensile force draws the medical device into the fistulous tract where it will be positioned with the longitudinal edges wedged into the damaged tissue. The particular shape with the presence of sharp edges on a solid body, which make the device which has been created non collapsible, in addition to any anti extrusion notches, allow the realization of the primary stability.

The conditions of an optimal biomechanical implant therefore fulfilled, the invented device will be integrated or reshaped by the patient's body with consequent repair of damaged tissue and healing of anal fistula in a proportion far more significant than those obtained with DM which, by their geometry do not favor the initial stability and the intimate contact.

This aim and these objectives, and others which will become apparent hereinafter are achieved by a medical device, specifically for the treatment of anal fistulas, made of a single element having the shape of a prism, said prism having two rectangular bases, a greater and a lesser, said bases having the same height and different lengths , whose perpendicular line represents the height of the same prism, said prism being formed by the two bases and two pairs of side faces, the pairs of side faces being respectively two parallel trapezoids and two rectangles inclined towards each other, said device having sharp edges along its entire length in the predetermined number given by its particular shape, said element being made of a biomaterial and having a solid body.

In the prior art it is possible to find some previous patents but they does not exceed certain problems, however, are superseded by this medical device.

The device U.S. -A2009/0125119 shows a medical product for treating fistulae comprising a "capping member" and a "plug member", hat and stem.

The shape of the "capping member" claimed by U.S. -A2009/0125119 (par. 0082) can be cubes, cuboids, tetrahedron, prism, pyramid, wedges and variations.

The device U.S. 2012 / 0035644 shows " barbs " along the axial axis of the device.

The diversity than is found in this: While the device U.S. 2012 / 0035644 "add" a sort of tips to the body of the device itself, the invention does not have anything in excess of his own body, but rather presents the " cuts " in the interior of the body itself which, by exploiting the force of gravity, in the case of extrusion, they open. The insertion of this invention in the human body, therefore, will be less difficult than the device referred to patent n. U.S. 2012/0035644, as it does not occur with a smooth body.

Further characteristics and advantages of the invention will become apparent from the detailed description of a specific but not exclusive embodiment, illustrated only by way of non-limitative examples in the accompanying drawings, in which:

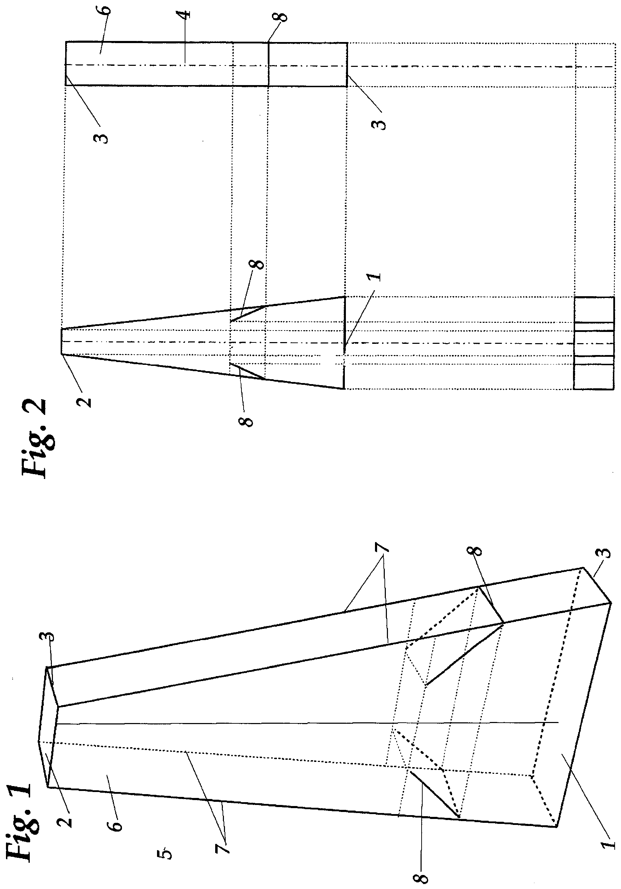

Fig 1 illustrates the medical device in axonometric perspective where there are two rectangular bases (1-2) having the same height (3), two mutually parallel trapezoidal faces (5), two rectangular faces inclined towards each other (6), presenting sharp edges (for its entire length (7).
Fig 2 illustrates the medical device, in orthogonal projection ;
Fig 3 illustrates the anti-extrusion notches in a closed position (3 a) and open position (3b) in the medical device ;

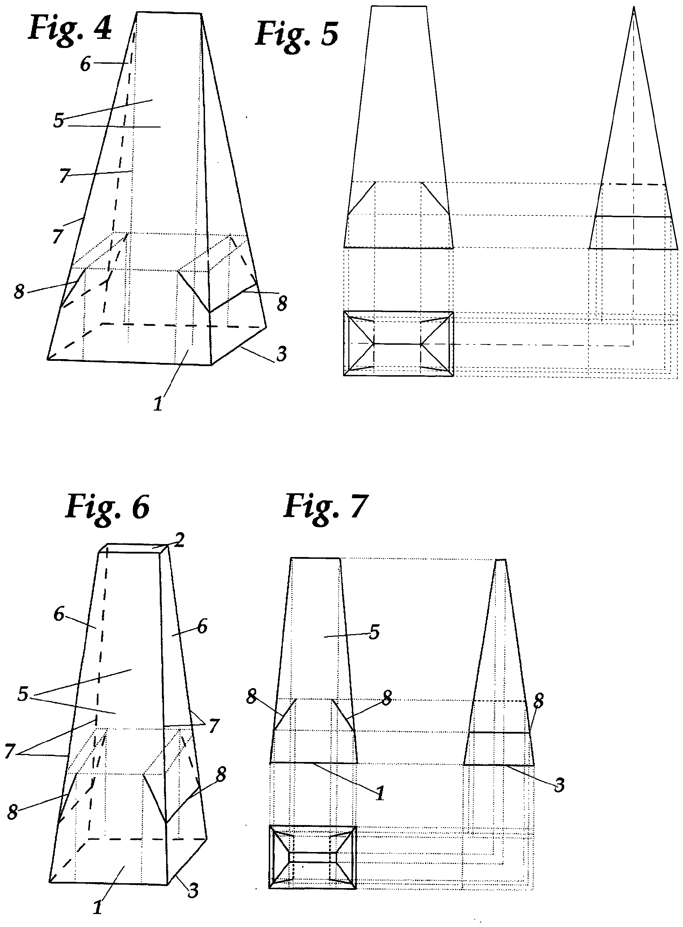

Figure 4 illustrates the medical device in the shape of a wedge in axonometric perspective with anti-extrusion notches (8) ;
Figure 5 illustrates the medical device in the shape of a wedge with anti-extrusion notches (8), in orthogonal projection ;
Figure 6 illustrates the medical device in the shape of an obelisk in axonometric perspective fitted with anti-extrusion notches (8) ;
Figure 7 illustrates the medical device in the shape of an obelisk with anti-extrusion notches (8), in orthogonal protection ;

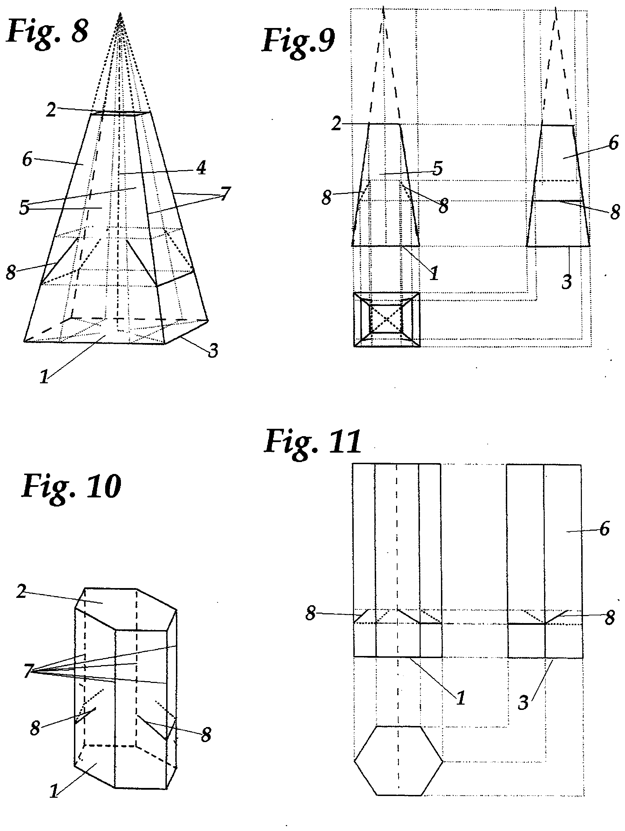

Figure 8 illustrates the medical device in the shape of a pyramid and truncated pyramid with anti-extrusion notches (8);
Figure 9 illustrates the medical device in the shape of a pyramid and truncated pyramid fitted anti-extrusion notches (8), in orthogonal protection ;
Figure 10 illustrates a medical device in the shape of a prism with a polygonal base , with anti-extrusion notches (8), in axonometric perspective not part of the invention;
Figure 11 illustrates the medical device in the shape of a prism with a polygonal base with anti-extrusion notches (8) , in orthogonal projection;
Figure 12 shows the rectum with anal fistulas;
Figure 13 illustrates the insertion of the medical device within the fistulous tract.

Note that anything discovered during the procedure of obtaining the patent which is found to be already known, is understood not to be claimed and subject of a disclaimer by these claims.

In each case the materials used and the dimensions constituting the invention may be more relevant according to specific requirements.

With reference to the above figures , it is indicated by figure n . 1, a medical device, for the treatment of anal fistulas, constituted by a single element having the shape of a prism. Said prism is formed by two rectangular bases, greater (1) and a lower (2), with the same height (3) and different lengths, where the perpendicular line (4) constitutes the height of the prism itself. Said prism, is formed by two bases and two pairs of lateral sides, respectively two trapezoids parallel to each other (5) and two rectangles inclined towards each other (6). The prism for its particular geometric shape presents sharp edges (7) along its entire length. Said device is made of biomaterial and is characterized by having a solid body so as not to be collapsible.

The device can be realized in an inert biomaterial, for example of the type known under the brand name " Vycril " of the company Johnson & Johnson , or bioactive agent , for example of the type known under the brand name " Strattice " Lifecell company.

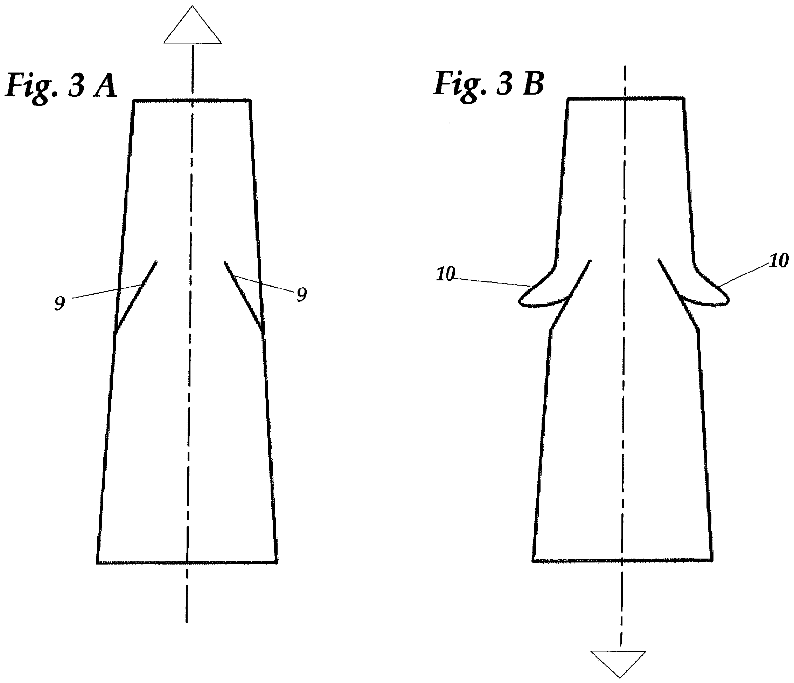

The measurements of the medical device are indicatively:
- The pair of trapezoidal side faces (5) and parallel with the larger base of predetermined size (from 1 mm to 20 mm) , the smaller base of predetermined size (from 0.5 mm to 19.9 mm) and predetermined height measurement (from 50 mm to 150 mm) ;
- The pair of rectangular side faces (6) between them with a sloping base of predetermined size (mm from 0.3 mm to 20 mm) and a height of predetermined size (50 mm by 250 mm). This device can also be characterized by the fact of having notches - fig. 3A and 3B (8-9-10) - in a predefined number from one to two pairs. The notches are full-thickness in the device , while preserving the body of the device and mirror one another (on the trapezoid) . Said notches are generated from the rectangular faces (6) inclined towards each other, are generated in parallel to the bases of the rectangular faces and inclined towards the smaller base of the prism (2) . Their function is anti-extrusion , remaining closed at implantation (9) and opening in the case of extrusion (10).

The figures illustrate alternative forms 4, 5, 6, 7, 8, 9, and are not exhaustive of the medical device invented , always taking into consideration that the technical characteristics of the invention are the presence of sharp edges , the solid body and anti-extrusion notches.

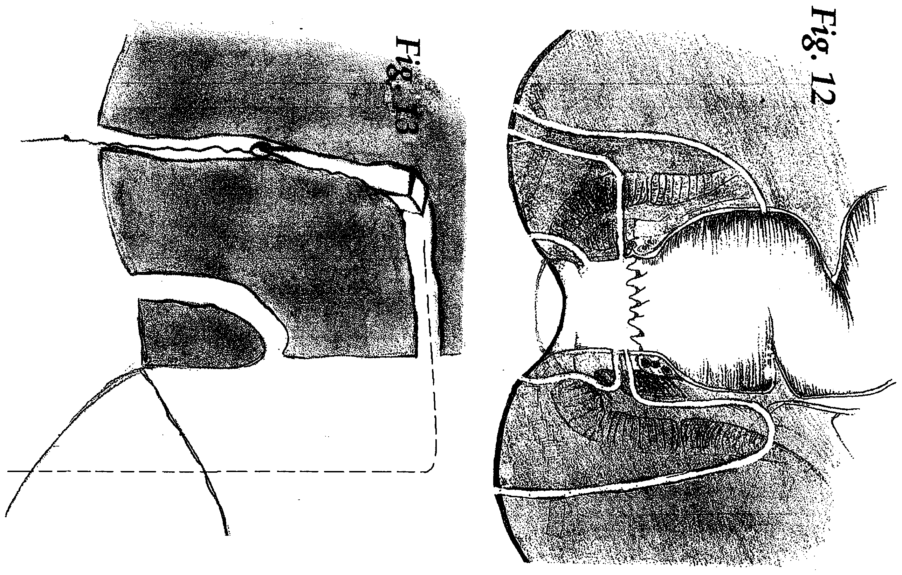

Figure 13 illustrates how the device is inserted into the fistulous tract. The distal tip of the device is rigidly fixed by means of a suture that , with axial tensile force draws the device itself inside the fistulous tract where it will be positioned with the longitudinal edges forming wedge pressure in the damaged tissue.

Different forms for performing certain different functions may not exist only in the illustrations, but may be present in many forms , also not illustrated.

The characteristics indicated as advantageous, convenient or the like may also be omitted or be replaced with equivalents.

## Claims

1. Medical device for the treatment of anal fistulas, consisting of a single element in the shape of a prism having two parallel rectangular bases (2), with same widths and different lengths, and two pair of side faces, one pair of mutually parallel trapezoids (5) and one pair of rectangles (6) inclined between them, that device having sharp edges (7) for its entire lenght, being made of a biomaterial and being solid.

2. Medical device of claim 1, which is **characterized by** the fact of being inert biomaterial or bioactive.

3. Medical device of claims 1 or 2, wherein the medical device has notches (8) incised into that device to a depth which safeguards the core of said device, being specular one another and being generated from the rectangular faces (6) inclined towards each other, those notches having a basis parallel to the rectangular bases and inclined towards the smaller end of the prism (2), those notches having anti-extrusion function, whereby they remain closed during surgical implantation and open only in case of extrusion.

## Patentansprüche

1. Medizinprodukt zur Behandlung von Analfisteln, bestehend aus einem einzelnen Element in Prismenform mit zwei rechteckigen parallelen Grundflächen (2) mit gleichen Breiten und unterschiedlichen Längen und zwei Seitenflächenpaaren, wovon ein Paar aus gegenseitigen parallelen Trapezen (5) und ein Paar aus Rechtecken (6) besteht, die untereinander geneigt sind; dieses Produkt weist auf der gesamten Länge scharfe Kanten (7) auf, da es aus Biomaterial gemacht und fest ist.

2. Medizinprodukt gemäß Anspruch 1, das **dadurch gekennzeichnet ist, dass** es aus trägem oder bioaktivem Biomaterial gemacht ist.

3. Medizinprodukt gemäß der Ansprüche 1 oder 2, bei dem das Medizinprodukt Einkerbungen (8) aufweist, die in diesem Produkt in einer Tiefe eingeschnitten wurden, durch die das Kernstück dieses Produkts geschützt ist, da sie spiegelgleich zueinander angeordnet sind und durch die zueinander hingeneigten rechteckigen Flächen (6) erzeugt werden, da diese Einkerbungen eine Grundfläche aufweisen, die parallel zu den rechteckigen und hin zum kleineren Ende des Prismas geneigten Grundflächen (2) ausgerichtet ist; diese Einkerbungen dienen als Schutz vor dem Herausziehen, sie bleiben daher während der chirurgischen Implantierung geschlossen und öffnen sich nur beim Herausziehen.

## Revendications

1. Dispositif médical pour le traitement des fistules anales, constitué d'un élément unique en forme de prisme ayant deux bases rectangulaires parallèles (2) de mêmes largeurs et de longueurs différentes et deux paires de faces latérales, une paire de trapèzes parallèles mutuels (5) et une paire de rectangles (6) inclinés entre eux, ce dispositif ayant des angles vifs (7) sur toute sa longueur, étant de corps solide et réalisé en biomatériau.

2. Dispositif médical conforme à la revendication 1, **caractérisé par le fait qu'**il est en biomatériau inerte ou bicactif.

3. Dispositif médical conforme aux revendications 1 ou 2, dans lequel le dispositif médical présente des encoches (8) incisées à une profondeur qui permet de conserver le noyau de ce dispositif, étant spéculaires l'une l'autre et générées par les faces rectangulaires (6) inclinées l'une vers l'autre, ces encoches ayant une base parallèle aux bases rectangulaires et étant inclinées vers la plus petite extrémité du prisme (2), ces encoches ayant une fonction anti-extrusion, restant par conséquent fermées au cours de l'implantation chirurgicale et ne s'ouvrant qu'en cas d'extrusion.
